# EUROPEAN PATENT SPECIFICATION

(11) **EP 2 155 159 B1**
(45) Date of publication and mention of the grant of the patent: **10.07.2019**
(21) Application number: 08767661.5
(22) Date of filing: 08.05.2008
(51) Int. Cl.: A61K 9/00, A61K 31/00

(54) **ROBUST RAPID DISINTEGRATION TABLET FORMULATION**
ROBUSTE TABLETTENFORMULIERUNG MIT SCHNELLER ZERSETZUNG
FORMULATION DE COMPRIMÉS ROBUSTES À DÉSINTÉGRATION RAPIDE

(30) Priority: 08.05.2007 US 928125 P
(43) Date of publication of application: 24.02.2010
(73) Proprietor: Hercules LLC, Wilmington, DE 19808 (US)
(72) Inventor: DURIG, Thomas, Chadds Ford, PA 19317 (US)
(74) Representative: Bülle, Jan
(86) International application number: PCT/US2008/005978
(87) International publication number: WO 2008/140772

(56) References cited:
- WO-A-03/032955
- WO-A-2006/072832
- US-A1- 2005 019 398

## Description

### Field of the Invention

A rapidly disintegrating, low friable tablet formulation is provided. The tablet formulation comprises: an ethylcellulose binder which is co-formulated with typical disintegrants and other common tablet aids such as fillers and tablet lubricants and flow aids. When tested, the tablet produced from the formulation exhibited a friability of about 5% or less and disintegrated in less than about 60 seconds.

### Background of the Invention

Rapidly disintegrating or fast dissolving tablets or oral dosage forms which are intended to rapidly break up and deliver an active ingredient in the oral cavity are gaining importance as a vehicle for administering nutraceutical and pharmaceutical active ingredients, especially in pediatric and geriatric populations. Generally such tablets are expected to have a very short residence time in the mouth. They should therefore disintegrate in less than 60 seconds and more ideally in less than 30 seconds when tested using a standard USP tablet disintegration test. In addition, the tablets should not provide an unpleasant mouthfeel, should not be excessively hard, in case they are chewed, and should also not have an unpleasant taste. From a manufacturing point of view, its is desirable that the tablet formulation yields mechanically robust tablets with low friability and is directly compressible, thus obviating time consuming coprocessing such as granulation steps, spray drying and freeze drying. A desirable friability can be defined as less than 1% and ideally less than 0.5% weight loss on attrition in a standard friabilator.

It is common to combine an active ingredient with soluble tablet fillers especially sugar alcohols (polyols) as many of these are not only soluble and compressible, but also provide a pleasant mouthfeel and may also provide some non-calorigenic sweetening effect. Frequently used polyols include mannitol, xylitol, sorbitol and lactiol. Other soluble tablet fillers include soluble sugars and starch derivatives such as sucrose, lactose, dextrose, maltodextrin, isomalt and polydextrose.

Typically, the soluble filler, such as a sugar alcohol, is further combined with a high level (5% or more by weight) of a hydrophilic, highly swellable disintegrant, such as cross-linked povidone (crospovidone), cross-linked sodium carboxymethyl cellulose (croscarmellose sodium), cross-linked sodium carboxymethyl starch (sodium starch glycollate) or low-substituted hydroxypropyl cellulose.

However when using such a formulation approach to make directly compressible tablets, it is difficult to simultaneously minimize disintegration times, friability and hygroscopicity. For example, disintegrants as a class are generally poorly compressible and have low tablet binding efficiency and are very hygroscopic. By increasing the amount of disintegrant to shorten disintegration time, the resultant tablets exhibit an increased hygroscopicity and a lower compactibility with increased friability. Moreover in direct compression and without additives such as binders, the majority of suitable tablet fillers, as named above tend to yield only marginally robust tablets. The addition of common water soluble tablet binders such as hydroxypropylcellulose (HPC), hydroxypropylmethyl cellulose (HPMC) or povidone (PVP) tends to be ineffective or result in longer disintegration times as the binder develops viscosity, gels and retards the breakup of the wet tablet.

In summary, common disadvantages of the above formulations are that due to the nature of materials used, these formulations tend to be hygroscopic and are also relatively poorly compactable, resulting in stability issues, high friabilities and relatively poor mechanical handling properties when compared to traditional directly-compressed immediate release tablets. As many drugs are poorly directly compressible, performance of the tablets worseness as the proportion of drug in the tablet increases. Therefore, special precautions need to be taken during mass handling and packaging of rapid disintegrating tablets to protect these tablets from excessive atmospheric moisture as well as excessive mechanical forces.

Several formulation strategies have been developed. In International Patent Application WO2006058250 A1, a rapidly disintegrating oral tablet formulation comprising a combination of two sugar alcohols which are co-processed to yield non-filamentous particles is disclosed. This mixture is combined with a third supplemental sugar alcohol, disintegrants and other components such as flow aids. The formulation produces rapidly disintegrating tablets with low friability.

In US Patent No. 6,284,272, the use of effervescent agents which result in rapid tablet break up on contact with the saliva has been taught. However, effervescent agents are generally a combination of acids and bases which destabilizes many active ingredients.

US Patent No. 5,631,023 teaches a rapid disintegrating tablet made from a lyophilized mixture of actives and excipients. By freeze drying the tablet formulation, the formulation is rendered amorphous and highly porous and thus extremely water-soluble. However, freeze drying is a specialized and time consuming process and the resultant tablets are extremely hygroscopic, necessitating additional manufacturing and packaging precautions for moisture control.

US Patent Application No. 20030138369A1 discloses a grade of calcium metasilicate with low particle aspect ratio and high oil or water absorption characteristics. According to the manufactures literature, these calcium metasilicate products function as co-agents in concert with other known disintegrants when used at levels up to 30% in fast dissolve formulations. However, the addition of calcium metasilicate may result in loss of tablet robustness and compactibility. A similar effect is found when calcium metasilicate is combined with dicalcium phosphate as illustrated in Example 6 of US Patent Application No. 20050244343A1. It should also be noted that calcium silicate in general is characterized by an alkaline surface pH which may be detrimental to the stability of alkali-labile drugs. Similar use of other inorganic additives such as titanium dioxide, silica or calcium carbonate in combination with a disintegrant and a sugar alcohol has also been disclosed, as in International Patent Application WO 2005110376 A3.

US Patent No. 5747068 teaches materials for use in readily dissolvable tablets include specially modified starches for fast dissolve tablets. Additionally, numerous rapidly disintegrating formulations for specific drugs can be found in the literature. For instance US Patent No. 5747068 additionally discloses dispersible tablets comprising fluoxetine and various disintegrants and soluble fillers.

US Patent No. 6,592,901 teaches a pharmaceutical dosage form composition composed of ethylcellulose that has an ethoxyl range lower limit of 49.6%, and a viscosity of less than 53 cps. This pharmaceutical dosage form is highly compressible and compactable and is capable of forming harder tablets or pellets with good release retardation. Further relevant prior art includes US 2005/019398 A1 which describes rapidly disintegrating tablet compositions.

There remains a need for a rapidly disintegrating mechanically robust low friable tablet formulation for fast and effective delivery of an active ingredient in the oral cavity.

### Brief Description of the Invention

The present invention, as defined by claim 1, relates to the use of ethylcellulose (EC) as a water-insoluble, inert tablet binder at levels of about 1 to about 20% by weight for rapid disintegrating tablets. EC is well known for its drug release retarding properties and use in non-disintegrating hydrophobic matrix tablets. However, the present invention relates to use of EC in a dosage form generally understood to require rapid disintegration and high solubility in water which is surprising, especially at the relatively high binder use of levels (e.g. 15% by weight of the tablet formulation).

More particularly, the present invention relates to a rapidly disintegrating, low friable tablet formulation comprising about 1 to 20% by weight of an ethylcellulose binder and about 2 to15% by weight of a disintegrant. The ethylcellulose binder has an ethoxyl content in the range of 44 to 54.9% and 5% solution viscosity in the range of about 3 to 200 cps in a 80:20 toluene: ethanol solvent blend. The disintegrant is selected from the group consisting of cross-linked povidone, sodium cross carmellose (cross-linked sodium carboxymethyl cellulose), sodium starch glycollate, low-substituted hydroxypropyl cellulose, and guar.

The present invention also relates to a method for producing a rapidly disintegrating, low friable tablet. The method comprising the steps of obtaining and blending an ethylcellulose binder, a disintegrant, and optionally a filler and a flow aid to produce a mixture. Coprocessing the mixture and compressing the coprocessed mixture to form the rapidly disintegrating, low friable tablet. The coprocessing may be accomplished by either co-milling, roller compacting or though the wet agglomeration of the mixture. A lubricant may be added to the mixture prior to compression into the tablet form.

The rapidly disintegrating, low friable tablet of the present invention also can be combined with at least one active pharmaceutical ingredient.

### Detailed Description of the Invention

It has been found that EC, a water-insoluble, hydrophobic cellulose ether, which is commonly used as a drug release retarding agent in barrier film coatings or hydrophobic non-disintegrating matrix tablets, can act as a synergistic tablet binder for rapidly disintegrating tablet formulations. EC, a non-hygroscopic, nonreactive tablet binder can readily be dry blended or co-processed (for example by co-milling or through use of agglomeration techniques including but not limited to roller compaction and wet granulation) with other formulation components to provide the combined attributes of fast disintegration (less than 60 seconds and frequently less than 20 seconds), relative inertness, near pH neutrality, ease of manufacturing by conventional direct compression tablet technology, and high tablet robustness as defined by low tablet friability (less than 1 % and frequently less than 0.5% friable by weight).

The invention also provides for tablet formulations with low hygroscopicity prior to compression into tablets and tablets also have very low hygroscopicity, not withstanding the fast dispersion in water. Typical moisture uptake is less than 2 % (on a dry weight basis) at 50% relative humidity and 25°C.

Ethylcellulose (EC) is a cellulose ether that is versatile with many uses. A preferred EC is described in US Patent No 6592901, which is incorporated herein by reference in its entirety. The following grade types of EC are commercially available from Hercules Incorporated:

| Type | Ethoxy Content (%) | Degree of Substitution (DS) |
|---|---|---|
| K | 45.0-47.3 | 2.22-2.41 |
| N | 48.0-49.5 | 2.46-2.58 |
| T | 49.6-51.5 | 2.58-2.73 |
| X | 50.5-52.5 | 2.65-2.81 |

Types K, N, and T of EC are used in food and food contact applications. More specifically, K and T are used for food and contact such as paper or paperboard in contact with food. N types were used as a binder or coating in pharmaceutical applications. Type X is used in inks and other industrial applications. While any grade of EC is of utility in this invention, the use of optimized direct compression grades such as high ethoxyl, low viscosity EC (T10 EC Pharm grade, available from Aqualon Division, a Business Unit of Hercules Incorporated), is especially preferred. This EC type combines high compressibility with good powder flow characteristics. Other commercially available grades of EC with lower ethoxyl and lower or higher viscosity (such as N7, N10, N14, N22, N50 and N100 Pharm grade EC, all available from Aqualon Division, a Business Unit of Hercules Incorporated), while possibly less effective than T10 EC Pharm grade, are also useful in the tablet formulations of the current invention.

It is well known in the art how to make EC. Normally, either chemical grade cotton linters or wood pulp is used to prepare EC. The sequence of chemical reactions is similar to that for methylation of cellulose. In commercial practice, sodium hydroxide concentrations of 50% or higher are used to prepare the alkali cellulose. Staged additions of solid sodium hydroxide during the reactions can be used to reduce side reactions. Ethyl chloride is added to the alkali cellulose in nickel-clad reactors at 90-150°C and 828 to 965 kPa (120 to 140 psi) for 6-12 hours. Diluents such as benzene or toluene can be used. At the end of the reaction, the volatiles such as ethyl chloride, diethyl ether, ethanol, and diluent are recovered and recycled. The ethylcellulose in solution is precipitated in the form of granules with further recovery of the carrier solvents. Washing with water completes the processing. Control of metallic impurities is important to achieve stability during storage. Anitoxidants can also be incorporated to inhibit loss of viscosity.

While any grade of EC is of utility in this invention , a preferred EC of use in the present invention has a higher ethoxyl content (greater than 49.6%) and simultaneously a low viscosity (less than 53 cps) and the average particle size is greater than 50 micrometers.

The preferred EC of use in the present invention has an ethoxyl content lower limit of 49.6%, preferably 49.8%, and more preferably 50.0%. The upper limit of the ethoxyl content of the EC is 54.88%, preferably 53.0% and more preferably and more preferably 52.0%. The viscosity of the EC is less than 53.0 cps, preferably less than 25 cps and more preferably less than about 17 cps, with a lower limit of about 3 cps.

The EC binder is co-formulated with typical disintegrants and other common tablet aids such as fillers and tablet lubricants and flow aids. Typical disintegrants include and may be selected from the group consisting of cross-linked povidone, sodium cross carmellose (cross-linked sodium carboxymethyl cellulose), sodium starch glycollate, low substituted hydroxypropyl cellulose, and guar. Low-substituted hydroxypropyl cellulose may be defined as having a hydroxypropoxyl content in the range of 5.0 to 16.0% by weight and an apparent average degree of polymerization in the range of 350 to 700. Low-substituted hydroxypropyl cellulose is disclosed in US Patent No. 6380381, incorporated herein by reference.

Suitable fillers include sucrose, lactose, dextrose, mannitol, xylitol, sorbitol, lactiol, maltodexrin, isomalt, polydextrose, starch and microcrystalline cellulose. Lubricants and flow aids include metal stearates, such as magnesium and calcium stearate, stearic acid, hydrogenated vegetable oils, poletheylene glycols, amino acids, stearyl fumarate, talc and colloidal silicone dioxide.

Other additives which are typically used in small amounts but are important for organoleptic enhancements include sweetners, flavors, tastemasking agents and colorants. Examples of sweeteners include sucralose, sodium saccharin, acesulfame K and aspartame. Examples of flavoring and tastemasking agents include peppermint, citrus and vanilla extracts, amino acid derivatives such as glutamic acid based derivatives. The above is not meant to be an exhaustive list of possible organoleptic enhancing aids.

Suitable use levels of EC are 1-20%, more preferably 3- 18% and most prerrably 5- 15%.

Suitable use levels for disintegrant are 2- 15%, more preferably 3-12% and most preferably 5-10%.

Suitable lubricant levels range from 0.1% to 2.5%. More preferably 0.25 to 2.0% and most preferably 0.5% to 1.5%.

While any grade of EC is of utility in this invention, the use of optimized direct compression grades such as high ethoxyl, low viscosity EC (T10 EC Pharm grade, available from Aqualon Division, a Business Unit of Hercules Incorporated), is especially preferred. This EC type combines high compressibility with good powder flow characteristics. Other commercially available grades of EC with lower ethoxyl and lower or higher viscosity (such as N7, N10, N14, N22, N50 and N100 Pharm grade EC, all available from Aqualon Division, a Business Unit of Hercules Incorporated), while possibly less effective than T10 EC Pharm grade, are also useful in the tablet formulations of the current invention.

The rapidly disintegrating, low friable tablet formulation of the present invention also can be combined with an active pharmaceutical ingredient or medicaments to prepare a formulation suitable for tableting or pelletizing. One or more active pharmaceutical ingredients may be combined in a single dosage form, depending on the chemical compatibility of the combined active ingredients and the ability to obtain the desired release rate from the dosage form for each active ingredient. The determination of the effective amount of the medicament per dosage unit is easily determined by skilled clinicians.

Representative types of active pharmaceutical ingredients include antacids, anti-inflammatory substances, anti-infectives, psychotropics, antimanics, anti-Parkinson's agents, anti-Alzheimer's agents, anti-Parkinson's agents, anti-Alzheimer's agents, stimulants, antihistamines, laxatives, decongestants, nutritional supplements, gastrointestinal sedatives, antidiarrheal preparations, antianginal drugs, antiarrhythmics, antihypertensive drugs, vasoconstrictors and migraine treatments, anticoagulants and anti-thrombotic drugs, analgesics, antipyretics, hypnotics, sedatives, antiemetics, anti-nauseants, anticonvulsants, neuromuscular drugs, hyper- and hypoglycemic agents, thyroid and antithyroid preparations, diuretics, antispasmodics, uterine relaxants, mineral and nutritional additives, anti-obesity drugs, anabolic drugs, erythropoietic drugs, antiasthmatics, expectorants, cough suppressants, mucolytics, antiuricemic drugs, topical analgesics, local anesthetics, polypeptide drugs, anti-HIV drugs, anti-diabetic agents, chemotherapeutic and anti-neoplastic drugs.

Examples of specific active pharmaceutical ingredients include aluminum hydroxide, prednisolone, dexamethasone, aspirin, acetaminophen, ibuprofen, isosorbide dinitrate, nicotinic acid, tetracycline, ampicillin, dexbrompheniramine, chlorpheniramine, albuterol pseudoephedrine, loratadine, theophylline, ascorbic acid, tocopherol, pyridoxine, methoclopramide, magnesium hydroxide, verapamil, procainamide hydrochloride, propranolol, captopril, ergotamine, furazepam, diazepam, lithium carbonate, insulin, furosemide, hydrochlorothiazide, guaiphenesin, dextromethorphan , benzocaine, ondansetron, cetrizine, dimenhydrinate, diphenhydramine, vitamin B12, famotidine, ranitidine, omerpazole, rabeprazole, esomeprazole, sildenafil, tadalafil, atorvastatin, simvastatin, valsartan, lorsartan, donepezil, galantamine, rivastigmine, carbidopa, levodopa, sertaline, pramipexole and ropinirole. It should be understood that any active pharmaceutical ingredients that is physically and chemically compatible with the EC of the present invention and other dosage form ingredients can be used in the present invention.

In the below mentioned examples, a cross linked CMC level of 5% by weight of the total formulation was found to be highly effective, yielding fast disintegration and low friability. It is however expected that depending on a formulation requirements e.g., drug solubility, load and desired disintegration time, the disintegrant level may vary between 2 and 1 5% by weight of the formulation. However, a distinguishing advantage of the current invention is that even though a formulation contains 25% of a hydrophobic drug, dimenhydrinate, the tablet none the less disintegrates in about 15 seconds while only requiring 5% by weight disintegrant - low levels of disintegrant in combination with a non-hygroscopic EC therefore decrease the hygroscopicity of the overall formulation.

Similarly, a T10 EC level of 5-10% by weight was found to be highly effective in reducing tablet friability and maintaining low disintegration time. However it is understood that depending on formulation characteristics, especially compactibility characteristics and mechanical properties and dose of drug, the level of EC binder may vary from 1 to 20% by weight of the total formulation.

The following examples will serve to illustrate the invention, parts and percentages being by weight unless otherwise indicated.

### Examples

### STANDARD METHODS FOR DETERMINING PROPERTIES

### Ethoxyl Content

In accordance with ASTM D4794, Ethoxyl content was determined by a Zeisel (sealed) tube method by reacting EC with hydriodic acid, liberating one mole of ethyl iodide for each mole of ethoxyl substitution on the cellulose chain. The ethyl iodide was then extracted with o-xylene and quantitated by gas chromatography using toluene as an internal standard. A typical set of apparatus, reagents and procedures for this test are listed below:

### Apparatus

1. Gas chromatograph, Perkin-Elmer 900, or equivalent equipped with thermal conductivity detector, chart recorder, and integrator.
2. Column 6'.times.1/8" stainless steel packed with 10% SP-2100 on 100/120 Supelcoport, Supelco, Inc., Bellefonte, Pa. Upon receipt, columns were conditioned overnight at 200°C.
3. Reacti-vials, 5 ml, equipped with mininert valves. (Pierce Chemical Co., #13223 and #10135).
4. Silli-Therm Heating Module, 110 v, 19791, Pierce Chemical Co., Rockford, III.
5 Reacti-Bar 21 (6) 19785, Pierce Chemical Co., Rockford, III.
6 Cover, stainless steel, fabricated to cover six (6) Reacti-Bar 21 units on the Silli-Therm Heating Module
7 Dispenser 0-5 ml, Labindustries Repipet, or equivalent. Syringe, 100 .mu.l, Hamilton 710 N or equivalent.
8 Syringe, Hamilton adjustable set to deliver 1.0 .mu.l injections.
9 Micro-set pipet adjusted to deliver 2.0 ml (Lancer product #8885-890007).
10 Balance: 0.0001 g. readability; 0.0002 g. accuracy.

### Reagents

1 lodoethane, reagent grade (ethyl iodide)
2 Toluene, reagent grade
3 O-xylene, reagent grade
4 Hydriodic acid, 57% solution in water.

**Gas Chromatoaraph and Integrator Parameters**

| | |
|---|---|
| Oven | 130°C. |
| Injection Port | 200°C. |
| Detector Current | 175 mA |
| Flow Rates: Helium | 30 ml./min. |
| Detector Temperature | 250°C |
| Attenuation | 3 |
| Chart Speed | 1.0 |
| Peak Width | 0.04 |
| Threshold | 4 |

Integrator parameters are given for Hewlett Packard Reporting Integrator Model 3390A.

### Procedure

1 Dried about 0.5 grams of sample in 105°C. oven for 1 hour.
2 Set heating block temperature to 150 °C.
3 Into a tared 5 ml reacti-vial, weighed 0.05-0.08 gram of cooled sample. Recorded weight to the nearest 0.0001 gram, samples were run in duplicate or triplicate.
4 Added 2 ml of hydriodic acid using a transfer pipet. Capped sample.
5 Added 2 ml of internal standard solution using the repipet dispenser or equivalent.
6 Immediately recapped vials with mininert valve tops and shook vials. Monitored block temperature at 180+/-5°C with a thermometer.
7 Placed vials into block and replaced metal cover. Kept samples behind safety shield while heating.
8 Maintained block temperature at 150+/-5°C for two hours.
9 Removed vials and allowed to cool to room temperature.
10 Shook each sample vigorously and allowed to stand for about 20 minutes.
11 Chromatographed 1.0 .mu.l of the upper solvent layer of each sample on the gas chromatograph.

### Viscosity

Viscosity was determined by preparing a 5% solution of EC in a toluene:ethanol (80:20) solvent mixture. Viscosity of the solution was measured using a Hercules Horizontal Capillary Viscometer (following ASTM D914-00, 33.1). The list of apparatus, reagents and procedures are described below.

### Apparatus

1. Balance, 0.1 g. accuracy.
2. Buret (optional) capable of delivering 111.8 ml.
3. Bath, constant temperature maintained at 25°C.
4. Eight oz., wide mouth, screw cap bottle with cap.
5. Cellophane or other suitable bottle cap liner.
6. Viscometer, Hercules Horizontal Capillary Viscometer--Calibrated to give viscosity readings in centipoise.
7. Thermometer, marked in 0.1°C subdivisions.
8. Shaker.

### Reagents

1. Ethyl Alcohol, SDA 2B-3 grade.
2. Toluene, meeting ASTM D 362 specification.
3. Toluene:Alcohol solvent, 80:20 by weight.

### Procedure

1. Determined the temperature of the 80:20 solvent to be used. The temperature of the solvent must be between 20 and 30°C if 111.8 ml. burette is to be used in this determination.
2. Weighed 5.0 g. of sample to the nearest 0.1 g.
3. Measured 111.8 ml. of 80:20 solvent from burette (the equivalent of 95.0 grams of solvent) into an 8-oz. bottle. Added the sample to the solvent, making an effort to disperse the sample and avoid lumping. Covered the neck of the bottle with a sheet of cellophane and applied the screw cap.
4. Placed the sample on a shaker and allowed it to shake until dissolution is complete.
5. Placed the bottle into a 25°C bath for 30 minutes and the solution was free of air bubbles.
6. With the viscometer in the raised position (reservoir vertical), filled the reservoir to the etched mark. Made sure that no air remained trapped in the sample. Placed a finger over the end of the capillary. Released brace and carefully lowered the viscometer to horizontal. (It was essential that the liquid was allowed to come to an equilibrium level before placing the finger over the end of the capillary and lowering it to the horizontal.)
7. Released the finger and measured the time for the liquid to flow from the first to the second mark in the capillary tube. Reported as time t.
8. Calculated the viscosity as follows: N=td/D where: N=viscosity, cps. t=time of flow for the sample d=density of sample solution at 25°C (0.86) D=density of the oil used for calibration of the viscometer.

Friability is measured by placing an accurately weighed sample of 20 tablets in the drum of a standard Roche-type friabilator and rotating the drum for 250 rotations. % Friability is then calculated as the % weight loss of the de-dusted tablets after rotation relative to the same sample of tablets prior to rotation in the friabilator.

Disintegration time is measured by placing 6 tablets into a standard USP disintegration apparatus without disc inserts. The tablets are then dipped and reciprocated in a pH 6.8 phosphate buffer solution (as defined in the USP) and carefully observed and timed. Disintegration time is recorded as the time where no discernible tablet core remains and all the pieces of the disintegrated tablet have fallen through the mesh screen of the disintegration cell. The temperature of the test solution is 37°C +/- 1°C.

### Tablet Formulation and Manufacture

For all examples, the various formulation components, with exception of magnesium stearate and stearic acid, were first dry blended in a Patterson-Kelly V-type blender for 15 minutes. Magnesium stearate and stearic acid were then added to the mixture through a 20 mesh screen, and the entire mass was then blended for another 3 minutes. Tablets were then directly compressed at 37 rpm on an instrumented Manesty Beta press, equipped with ¼" standard concave tooling, except where larger tooling is indicated. A target weight of 100 mg was set, except where a different weight is indicated. Tablets were compressed at 5 kN and approximately 8 kN of compressive force for examples using ¼" standard concave tooling. For larger tooling, 15, 20 and 25 kN compressive force was used. For larger tooling 15, 20 and 25 kN compressive force was used. Tablet crushing strength was determined by diametrically compressing tablets using a Key Pharmatest HT500S hardness tester.

### Comparative Example 1.

A 500 gram batch of dry blended powder without EC was prepared and then tableted into 100 mg. tablets as a control formulation:

| | Parts by weight |
|---|---|
| Dimenhydrinate | 25 |
| Granular mannitol | 69.25 |
| Croscarmellose sodium | 5 |
| Stearic acid | 0.5 |
| Magnesium Stearate | 0.25 |

Table 1. Resultant crushing strength, friability and disintegration times for the control formulation in example 1. Tablets were made at 5 kN and 8 kN compression force using a rotary tablet press.

**Table 1**

| **Attribute** | **5kN Compression Force** | **8kN Compression Force** |
|---|---|---|
| Crushing strength (kP) | 0.8 | 1.0 |
| Friability (%) | 33%* | 18%* |
| Disintegration Time (secs.) | 14 | 15 |

| | | |
|---|---|---|
| *Tablets capped (delaminated) on friability testing. | | |

The combination of mannitol and croscarmellose were able to provide relatively fast disintegration of a tablet comprising 25% of a low soluble drug, dimenhydrinate. However, tablet friability was unacceptably high at 9% weight loss.

### Comparative Example 2.

A 500 gram batch of dry blended powder was prepared as above, however a low viscosity water soluble binder Klucel® EXF Pharm hydroxypropyl cellulose, available from Aqualon Division, a Business Unit of Hercules Incorporated was added and tableted into 100 mg. tablets :

| | Parts by weight |
|---|---|
| Dimenhydrinate | 25 |
| Hydroxypropyl cellulose | 15 |
| Granular mannitol | 54.25 |
| Croscarmellose | 5 |
| Stearic acid | 0.5 |
| Magnesium Stearate | 0.25 |

Table 2. Resultant crushing strength, friability and disintegration times for the control formulation in example 2. Tablets were made at 5 kN and 8 kN compression force using a rotary tablet press.

**Table 2**

| **Attribute** | **5kN Compression Force** | **8kN Compression Force** |
|---|---|---|
| Crushing strength (kP) | 3.24 | 4.72 |
| Friability (%) | 0.16 | 0 |
| Disintegration Time (secs.) | 185 | 200 |

Addition of hydroxypropyl cellulose was very effective in lowering friability and enhancing tablet strength but disintegration times in excess of 180 seconds resulted.

### Example 1.

A 500 gram batch of dry blended powder was prepared as above in comparative example 2, however in place hydroxypropyl cellulose, water insoluble T10 Pharm EC, available from Aqualon Division, a Business Unit of Hercules Incorporated, was substituted in the composition and tableted into 100 mg. tablets:

| | Parts by weight |
|---|---|
| Dimenhydrinate | 25 |
| T10 Pharm EC | 15 |
| Granular mannitol | 54.25 |
| Croscarmellose | 5 |
| Stearic acid | 0.5 |
| Magnesium Stearate | 0.25 |

Table 3. Resultant crushing strength, friability and disintegration times for the control formulation in example 1. Tablets were made at 5 kN and 8 kN compression force using a rotary tablet press.

**Table 3**

| **Attribute** | **5kN Compression Force** | **8kN Compression Force** |
|---|---|---|
| Crushing strength (kP) | 2.1 | 3.3 |
| Friability (%) | 0.3% | 5% |
| Disintegration Time (secs.) | 15 | 22 |

Substitution of hydroxypropyl cellulose with T10 Pharm EC was effective in maintaining the low friability and improved tablet strength relative to control, and was also effective in maintaining a rapid disintegration time of less than 30 seconds.

### Example 2.

A 500 gram batch of dry blended powder was prepared as above in example 1, however in place of 15% water insoluble T10 Pharm EC only 10% of T10 Pharm EC was included and tableted into 100 mg. tablets:

| | Parts by weight |
|---|---|
| Dimenhydrinate | 25 |
| T10 Pharm EC | 10 |
| Granular mannitol | 59.25 |
| Croscarmellose | 5 |
| Stearic acid | 0.5 |
| Magnesium Stearate | 0.25 |

Table 4. Resultant crushing strength, friability and disintegration times for the control formulation in example 2. Tablets were made at 5 and 8 kN compression force using a rotary tablet press.

**Table 4**

| **Attribute** | **5kN Compression Force** | **8kN Compression Force** |
|---|---|---|
| Crushing strength (kP) | 1.66 | 2.68 |
| Friability (%) | 3.5 | 0.1 |
| Disintegration Time (secs.) | 14 | 14 |

Reducing the EC component from 15% to 10% did not compromise low tablet friability while providing rapid disintegration times similar to those of the control.

### Example 3.

A 500 gram batch of dry blended powder was prepared as above in example 2, however in place of 10% water insoluble T10 Pharm EC only 5% of T10 Pharm EC was included and tableted into 100 mg. tablets:

| | Parts by weight |
|---|---|
| Dimenhydrinate | 25 |
| T10 Pharm EC | 5 |
| Granular mannitol | 64.45 |
| Croscarmellose | 5 |
| Stearic acid | 0.5 |
| Magnesium Stearate | 0.25 |

Table 5. Resultant crushing strength, friability and disintegration times for the control formulation in example 3. Tablets were made at 5 kN and 8 kN compression force using a rotary tablet press.

**Table 5**

| **Attribute** | **5kN Compression Force** | **8kN Compression Force** |
|---|---|---|
| Crushing strength (kP) | 1.38 | 2.3 |
| Friability (%) | 2.76 | 0.6 |
| Disintegration Time (secs) | 18 | 17 |

Reducing the EC component from 10% to 5% again allowed significant improvements in tablet friability relative to the control in comparative example 1, while maintaining rapid disintegration times below 30 seconds.

### Example 4.

A 500 gram batch of dry blended powder was prepared as above in example 2, however in place of dimenhydrinate, 25% directly compressible (pre-granulated) acetaminophen granulation was included. The tablet weight was increased from 100 mg used in comparative examples 1-2 and examples 1-3 to 120 mg.:

| | Parts by weight |
|---|---|
| Directly Compressible Acetaminophen | 25 |
| T10 Pharm EC | 5 |
| Granular mannitol | 64.45 |
| Croscarmellose | 5 |
| Stearic acid | 0.5 |
| Magnesium Stearate | 0.25 |

Table 6. Resultant crushing strength, friability and disintegration times for the control formulation in example 2. Tablets were made at 5 kN, 8 kN and 15kN compression force using a rotary tablet press.

**Table 6**

| **Attribute** | **5kN Compression Force** | **8kN Compression Force** | **15kN Compression Force** |
|---|---|---|---|
| Crushing strength (kP) | 2.0 | 3.5 | 3.9 |
| Friability (%) | 0.8 | 0.2 | 0.1 |
| Disintegration Time (secs) | 14 | 17.5 | 15.2 |

Acetaminophen is commonly known as a poorly compressible drug. The data show that the formulation system is is able to accommodate a series of different physico-chemical drug characteristics while maintaining low friability and rapid disintegration.

### Example 5.

A 500 gram batch of dry blended powder was prepared as above in example 4, however in addition to granular mannitol, 10% liquid sorbitol was added after initial dry blending of drug, ethylcellulose, mannitol, croscarmellose. The liquid sorbitol (70% sorbitol in 30% water) was added gradually while mixing to form a homogenous, "dry to the touch", free flowing powder. The amount of ethylcellulose and croscarmelose were also increased. After lubricant addition the 120 mg tablets were then compressed as in example 4.

| | Parts by weight |
|---|---|
| Directly Compressible Acetaminophen | 25 |
| T10 Pharm EC | 10 |
| Granular mannitol | 47.25 |
| Liquid sorbitol (70% sorbitol, 30% water) | 10 |
| Croscarmellose | 7 |
| Stearic acid | 0.5 |
| Magnesium Stearate | 0.25 |

**Table 7**

| **Attribute** | **3kN Compression Force** | **5kN Compression Force** | **8kN Compression Force** |
|---|---|---|---|
| Crushing strength (kP) | 2.0 | 3.4 | 4.4 |
| Friability (%) | 1.18 | 0.08 | 0.17 |
| Disintegration Time (sees) | 35.9 | 42.2 | 45.4 |

### Example 6.

A 500 gram batch of dry blended powder was prepared as above in example 5, however in place of liquid sorbitol, 10% spray dried sorbitol was used. The tablets were compressed using 5/8" round troche tooling with circular elevation in the center of the punch face, such that the center of the tablet was thinner than the perimeter of the tablet. Tablet target weight was 900 mg and tablets were compressed at 15,20 and 25 kN.

| | Parts by weight |
|---|---|
| Directly Compressible Acetaminophen | 25 |
| T10 Pharm EC | 10 |
| Granular mannitol | 47.25 |
| Spray dried sorbitol | 10 |
| Croscarmellose | 7 |
| Stearic acid | 0.5 |
| Magnesium Stearate | 0.25 |

**Table 8**

| **Attribute** | **15kN Compression Force** | **20kN Compression Force** | **25kN Compression Force** |
|---|---|---|---|
| Crushing strength (kP) | 4.7 | 6.5 | 6.4 |
| Friability (%) | 0.9 | 0.31 | 0.12 |
| Disintegration Time (secs) | 40.7 | 50.7 | 55.9 |

Examples 5 and 6 show the versatility of the system with regard to different tablet sizes and geometries, as well as inclusion of a diverse range of and physical forms of sugar alcohols and ingredients.

## Claims

1. A rapidly disintegrating, low friable tablet formulation **for producing a rapidly disintegrating, low friable tablet exhibiting a friability of 5% by weight or less and disintegrating in less than 60 seconds, the formulation** comprising:
a) 1 to 20% by weight of an ethylcellulose binder having an ethoxyl content in the range of 44 to 54.9% and 5% solution viscosity in the range 0.003 to 0.2 Pa s (3 to 200 cps) in a 80:20 toluene: ethanol solvent blend, and
b) 2 to 15% by weight of a disintegrant selected from the group consisting of cross-linked povidone, sodium cross carmellose (cross-linked sodium carboxymethyl cellulose), sodium starch glycollate, low-substituted hydroxypropyl cellulose, and guar.

2. The rapidly disintegrating, low friable tablet formulation of claim 1, wherein ethylcellulose binder comprises 3 to 18% by weight of the tablet formulation.

3. The rapidly disintegrating, low friable tablet formulation of claim 1, wherein ethylcellulose binder has an ethoxyl content lower limit of 49.6%.

4. The rapidly disintegrating, low friable tablet formulation of claim 1, wherein ethylcellulose binder has an ethoxyl content upper limit of 51.0%.

5. The rapidly disintegrating, low friable tablet formulation of claim 1, wherein ethylcellulose binder has 5% solution viscosity less than 0.053 Pa s (53.0 cps) in a 80:20 toluene: ethanol solvent blend.

6. The rapidly disintegrating, low friable tablet formulation of claim 1, wherein the disintegrant comprises 3-12% by weight of the tablet formulation.

7. The rapidly disintegrating, low friable tablet formulation of claim 1, wherein the rapidly disintegrating, low friable tablet formulation further comprises a filler wherein the filler is selected from the group consisting of sucrose, lactose, dextrose, mannitol, xylitol, sorbitol, lactiol, maltodexrin, isomalt, polydextrose, starch and microcrystalline cellulose.

8. The rapidly disintegrating, low friable tablet formulation of claim 1, wherein the rapidly disintegrating, low friable tablet formulation further comprises a lubricant wherein the lubricant comprises 0.1 to 2.5% by weight of the tablet formulation.

9. The rapidly disintegrating, low friable tablet formulation of claim 8, wherein the lubricant is selected from the group consisting of metal stearates, such as magnesium and calcium stearate, stearic acid, hydrogenated vegetable oils, polyethylene glycol, amino acid and stearyl fumarate.

10. The rapidly disintegrating, low friable tablet formulation of claim 1, wherein the rapidly disintegrating, low friable tablet formulation further comprises a flow aid wherein the flow aid is selected from the group consisting of talc and colloidal silicone dioxide.

11. The rapidly disintegrating, low friable tablet formulation of claim 1, wherein the rapidly disintegrating, low friable tablet formulation further comprises an active pharmaceutical ingredient.

12. The rapidly disintegrating, low friable tablet formulation of claim 11, wherein the active pharmaceutical ingredient is selected from the group consisting of aluminum hydroxide, prednisolone, dexamethasone, aspirin, acetaminophen, ibuprofen, isosorbide dinitrate, nicotinic acid, tetracycline, ampicillin, dexbrompheniramine, chlorpheniramine, albuterol pseudoephedrine, loratadine, theophylline, ascorbic acid, tocopherol, pyridoxine, methoclopramide, magnesium hydroxide, verapamil, procainamide hydrochloride, propranolol, captopril, ergotamine, furazepam, diazepam, lithium carbonate, insulin, furosemide, hydrochlorothiazide, guaiphenesin, dextromethorphan, benzocaine, ondansetron, cetrizine, dimenhydrinate, diphenhydramine, vitamin B12, famotidine, ranitidine, omepazole, rabeprazole, esomeprazole, sildenafil, tadalafil, atorvastatin, simvastatin, valsartan, lorsartan, donepezil, galantamine, rivastigmine, carbidopa, levodopa, sertaline, pramipexole and ropinirole.

13. A method for producing a rapidly disintegrating, low friable tablet **exhibiting a friability of 5% by weight or less and disintegrating in less than 60 seconds, the method** comprising the steps of:
a) obtaining and blending
i) 1 to 20% by weight of an ethylcellulose binder having an ethoxyl content in the range of 44 to 54.9% and 5% solution viscosity in the range of 0.003 to 0.2 Pa s (3 to 200 cps) in a 80:20 toluene: ethanol solvent blend,
ii) 2 to 15% by weight of a disintegrant selected from the group consisting of cross-linked povidone, sodium cross carmellose (cross-linked sodium carboxymethyl cellulose), sodium starch glycollate, low-substituted hydroxypropyl cellulose, and guar; and
iii) optionally a filler and a flow aid
to produce a mixture; and
b) compressing the mixture to form the rapidly disintegrating, low friable tablet.

14. The method for producing a rapidly disintegrating, low friable tablet of claim 13, further comprising the step of coprocessing the mixture prior to compressing the mixture to form the rapidly disintegrating, low friable tablet wherein the coprocessing step is selected from the group consisting of co-milling, roller compacting and wet agglomeration.

15. The method for producing a rapidly disintegrating, low friable tablet of claim 14, further comprising the step of adding a lubricant to the coprocessed mixture.

## Patentansprüche

1. Schnell zerfallende, schwach bröckelige Tablettenformulierung zur Herstellung einer schnell zerfallenden, schwach bröckeligen Tablette, die eine Bröckeligkeit von 5 Gew.-% oder weniger aufweist, und die in weniger als 60 Sekunden zerfällt, wobei die Formulierung umfasst:
a) 1 bis 20 Gew.-% eines Ethylcellulosebindemittels mit einem Ethoxylgehalt im Bereich von 44 bis 54,9% und 5%-Lösungsviskosität im Bereich von 0,003 bis 0,2 Pa s (3 bis 200 cps) in einem 80:20 Toluol:Ethanol-Lösungsmittelgemisch, und
b) 2 bis 15 Gew.-% eines Sprengmittels, ausgewählt aus der Gruppe bestehend aus vernetztem Povidon, Natrium-Cross-Carmellose (vernetzter Natriumcarboxymethylcellulose), Natriumstärkeglycollat, schwach substituierter Hydroxypropylcellulose und Guar.

2. Schnell zerfallende, schwach bröckelige Tablettenformulierung nach Anspruch 1, wobei das Ethylcellulosebindemittel 3 bis 18 Gew.-% der Tablettenformulierung ausmacht.

3. Schnell zerfallende, schwach bröckelige Tablettenformulierung nach Anspruch 1, wobei das Ethylcellulosebindemittel eine untere Grenze des Ethoxylgehalts von 49,6% aufweist.

4. Schnell zerfallende, schwach bröckelige Tablettenformulierung nach Anspruch 1, wobei das Ethylcellulosebindemittel eine obere Grenze des Ethoxylgehalts von 51,0% aufweist.

5. Schnell zerfallende, schwach bröckelige Tablettenformulierung nach Anspruch 1, wobei das Ethylcellulosebindemittel eine 5%-Lösungsviskosität von weniger als 0,053 Pa s (53,0 cps) in einem 80:20 Toluol:Ethanol-Lösungsmittelgemisch aufweist.

6. Schnell zerfallende, schwach bröckelige Tablettenformulierung nach Anspruch 1, wobei das Sprengmittel 3 bis 12 Gew.-% der Tablettenformulierung ausmacht.

7. Schnell zerfallende, schwach bröckelige Tablettenformulierung nach Anspruch 1, wobei die schnell zerfallende, schwach bröckelige Tablettenformulierung zudem einen Füllstoff umfasst, wobei der Füllstoff ausgewählt ist aus der Gruppe bestehend aus Saccharose, Lactose, Dextrose, Mannit, Xylit, Sorbit, Lactiol, Maltodextrin, Isomalt, Polydextrose, Stärke und mikrokristalliner Cellulose.

8. Schnell zerfallende, schwach bröckelige Tablettenformulierung nach Anspruch 1, wobei die schnell zerfallende, schwach bröckelige Tablettenformulierung zudem ein Gleitmittel umfasst, wobei das Gleitmittel 0,1 bis 2,5 Gew.-% der Tablettenformulierung ausmacht.

9. Schnell zerfallende, schwach bröckelige Tablettenformulierung nach Anspruch 8, wobei das Gleitmittel ausgewählt ist aus der Gruppe bestehend aus Metallstearaten, wie Magnesium- und Calciumstearat, Stearinsäure, hydrierten Pflanzenölen, Polyethylenglykol, Aminosäure und Stearylfumarat.

10. Schnell zerfallende, schwach bröckelige Tablettenformulierung nach Anspruch 1, wobei die schnell zerfallende, schwach bröckelige Tablettenformulierung zudem ein Fließhilfsmittel umfasst, wobei das Fließhilfsmittel ausgewählt ist aus der Gruppe bestehend aus Talk und kolloidalem Silikondioxid.

11. Schnell zerfallende, wenig bröckelige Tablettenformulierung nach Anspruch 1, wobei die schnell zerfallende, wenig bröckelige Tablettenformulierung zudem einen pharmazeutischen Wirkstoff umfasst.

12. Schnell zerfallende, schwach bröckelige Tablettenformulierung nach Anspruch 11, wobei der pharmazeutische Wirkstoff ausgewählt ist aus der Gruppe bestehend aus Aluminiumhydroxid, Prednisolon, Dexamethason, Aspirin, Acetaminophen, Ibuprofen, Isosorbiddinitrat, Nicotinsäure, Tetracyclin, Ampicillin, Dexbrompheniramin, Chlorpheniramin Albuterol-Pseudoephedrin, Loratadin, Theophyllin, Ascorbinsäure, Tocopherol, Pyridoxin, Methoclopramid, Magnesiumhydroxid, Verapamil, Procainamid-Hydrochlorid, Propranolol, Captopril, Ergotamin, Furazepam, Diazepam, Lithiumcarbonat, Insulin, Furosemid, Hydrochlorthiazid, Guaiphenesin, Dextromethorphan, Benzocain, Ondansetron, Cetrizin, Dimenhydrinat, Diphenhydramin, Vitamin B12, Famotidin, Ranitidin, Omepazol, Rabeprazol, Esomeprazol, Sildenafil, Tadalafil, Atorvastatin, Simvastatin, Valsartan, Lorsartan, Donepezil, Galantamin, Rivastigmin, Carbidopa, Levodopa, Sertalin, Pramipexol und Ropinirol.

13. Verfahren zur Herstellung einer schnell zerfallenden, schwach bröckeligen Tablette, die eine Bröckligkeit von 5 Gew.-% oder weniger aufweist, und die in weniger als 60 Sekunden zerfällt, wobei das Verfahren die Schritte umfasst:
a) Erhalten und Mischen von
i) 1 bis 20 Gew.-% eines Ethylcellulosebindemittels mit einem Ethoxylgehalt im Bereich von 44 bis 54,9% und 5%-Lösungsviskosität im Bereich von 0,003 bis 0,2 Pa s (3 bis 200 cps) in einem 80:20 Toluol:Ethanol-Lösungsmittelgemisch,
ii) 2 bis 15 Gew.-% eines Sprengmittels, ausgewählt aus der Gruppe bestehend aus vernetztem Povidon, Natrium-Cross-Carmellose (vernetzter Natriumcarboxymethylcellulose), Natriumstärkeglycollat, schwach substituierter Hydroxypropylcellulose und Guar; und
iii) gegebenenfalls eines Füllstoffs und eines Fließhilfsmittels
zur Herstellung eines Gemischs; und
b) Pressen des Gemischs, um die schnell zerfallende, schwach bröckelige Tablette herzustellen.

14. Verfahren zur Herstellung einer schnell zerfallenden, schwach bröckeligen Tablette nach Anspruch 13, zudem umfassend den Schritt der gemeinsamen Verarbeitung des Gemischs vor dem Pressen des Gemischs, um die schnell zerfallende, schwach bröckelige Tablette herzustellen, wobei der gemeinsame Verarbeitungsschritt ausgewählt ist aus der Gruppe bestehend aus gemeinsamem Mahlen, Walzenverdichtung und Nassagglomeration.

15. Verfahren zur Herstellung einer schnell zerfallenden, schwach bröckeligen Tablette nach Anspruch 14, zudem umfassend den Schritt des Zugebens eines Gleitmittels zu dem gemeinsam verarbeiteten Gemisch.

## Revendications

1. Formule de comprimé faiblement friable à désagrégation rapide destinée à produire un comprimé faiblement friable à désagrégation rapide présentant une friabilité de 5 % en masse ou moins et se désagrégeant en moins de 60 secondes, la formule comprenant :
a) 1 à 20 % en masse d'un agent liant de type éthylcellulose présentant une teneur en éthoxyles comprise dans l'intervalle allant de 44 à 54,9 % et une viscosité de solution à 5 % comprise dans l'intervalle allant de 0,003 à 0,2 Pa.s (3 à 200 cps) dans un mélange de solvants toluène/ éthanol 80:20, et
b) 2 à 15 % en masse d'un agent délitant choisi dans le groupe constitué par les suivants : povidone réticulée, croscarmellose sodique (carboxyméthylcellulose sodique réticulée), glycolate d'amidon sodique, hydroxypropylcellulose faiblement substitué et gomme de guar.

2. Formule de comprimé faiblement friable à désagrégation rapide selon la revendication 1, où l'agent liant de type éthylcellulose constitue 3 à 18 % en masse de la formule de comprimé.

3. Formule de comprimé faiblement friable à désagrégation rapide selon la revendication 1, où l'agent liant de type éthylcellulose présente une limite basse de teneur en éthoxyles de 49,6 %.

4. Formule de comprimé faiblement friable à désagrégation rapide selon la revendication 1, où l'agent liant de type éthylcellulose présente une limite haute de teneur en éthoxyles de 51,0 %.

5. Formule de comprimé faiblement friable à désagrégation rapide selon la revendication 1, où l'agent liant de type éthylcellulose présente une viscosité de solution à 5 % inférieure à 0,053 Pa.s (53,0 cps) dans un mélange de solvants toluène/éthanol 80:20.

6. Formule de comprimé faiblement friable à désagrégation rapide selon la revendication 1, où l'agent délitant constitue 3 à 12 % en masse de la formule de comprimé.

7. Formule de comprimé faiblement friable à désagrégation rapide selon la revendication 1, où la formule de comprimé faiblement friable à désagrégation rapide comprend en outre une charge, où la charge est choisie dans le groupe constitué par les suivantes : sucrose, lactose, dextrose, mannitol, xylitol, sorbitol, lactiol, maltodexrine, isomalt, polydextrose, amidon et cellulose microcristalline.

8. Formule de comprimé faiblement friable à désagrégation rapide selon la revendication 1, où la formule de comprimé faiblement friable à désagrégation rapide comprend en outre un lubrifiant, où le lubrifiant comprend 0,1 à 2,5 % en masse de la formule du comprimé.

9. Formule de comprimé faiblement friable à désagrégation rapide selon la revendication 8, où le lubrifiant est choisi dans le groupe constitué par les suivants : stéarates métalliques, tels que stéarate de magnésium et de calcium, acide stéarique, huiles végétales hydrogénées, polyéthylène glycol, acide aminé et fumarate de stéaryle.

10. Formule de comprimé faiblement friable à désagrégation rapide selon la revendication 1, où la formule de comprimé faiblement friable à désagrégation rapide comprend en outre un auxiliaire rhéologique, où l'auxiliaire rhéologique est choisi dans le groupe constitué par le talc et le dioxyde de silicium colloïdal.

11. Formule de comprimé faiblement friable à désagrégation rapide selon la revendication 1, où le comprimé faiblement friable à désagrégation rapide comprend en outre un principe actif pharmaceutique.

12. Formule de comprimé faiblement friable à désagrégation rapide selon la revendication 11, où le principe actif pharmaceutique est choisi dans le groupe constitué par les suivants : hydroxyde d'aluminium, prednisolone, dexaméthasone, aspirine, acétaminophène, ibuprofène, dinitrate d'isosorbide, acide nicotinique, tétracycline, ampicilline, dexbromphéniramine, chlorphéniramine, albutérol pseudoéphédrine, loratadine, théophylline, acide ascorbique, tocophérol, pyridoxine, méthoclopramide, hydroxyde de magnésium, vérapamil, chlorhydrate de procaïnamide, propranolol, captopril, ergotamine, furazépam, diazépam, carbonate de lithium, insuline, furosémide, hydrochlorothiazide, guaiphénésine, dextrométhorphane, benzocaïne, ondansétron, cétrizine, dimenhydrinate, diphenhydramine, vitamine B12, famotidine, ranitidine, omépazole, rabéprazole, ésoméprazole, sildénafil, tadalafil, atorvastatine, simvastatine, valsartan, lorsartan, donépézil, galantamine, rivastigmine, carbidopa, lévodopa, sertaline, pramipexole et ropinirole.

13. Procédé de production d'un comprimé faiblement friable à désagrégation rapide présentant une friabilité de 5 % en masse ou moins et se désagrégeant en moins de 60 secondes, le procédé comprenant les étapes consistant à :
a) obtenir et mélanger
i) 1 à 20 % en masse d'un agent liant de type éthylcellulose présentant une teneur en éthoxyles comprise dans l'intervalle allant de 44 à 54,9 % et une viscosité de solution à 5 % comprise dans l'intervalle allant de 0,003 à 0,2 Pa.s (3 à 200 cps) dans un mélange de solvants toluène/ éthanol 80:20,
ii) 2 à 15 % en masse d'un agent délitant choisi dans le groupe constitué par les suivants : povidone réticulée, croscarmellose sodique (carboxyméthylcellulose sodique réticulée), glycolate d'amidon sodique, hydroxypropylcellulose faiblement substitué et gomme de guar ; et
iii) éventuellement une charge et un auxiliaire rhéologique pour produire un mélange ; et
b) compresser le mélange pour former le comprimé faiblement friable à désagrégation rapide.

14. Procédé de production d'un comprimé faiblement friable à désagrégation rapide selon la revendication 13, comprenant en outre l'étape de co-transformation du mélange avant de compresser le mélange pour former le comprimé faiblement friable à désagrégation rapide, où l'étape de co-transformation est choisie dans le groupe constitué par les suivantes : co-broyage, compactage au rouleau et agglomération humide.

15. Procédé de production d'un comprimé faiblement friable à désagrégation rapide selon la revendication 14, comprenant en outre l'étape consistant à ajouter un lubrifiant au mélange co-transformé.
